**Europäisches Patentamt**

(19) **European Patent Office**

**Office européen des brevets**

(11) Publication number : **0 306 465 B1**

(12) **EUROPEAN PATENT SPECIFICATION**

(45) Date of publication of patent specification :
22.07.92 Bulletin 92/30

(21) Application number : **88850284.6**

(22) Date of filing : **26.08.88**

(51) Int. Cl.⁵ : **B01J 2/30,** B01J 2/14,
// A61K9/16

(54) **Granular product (III).**

(30) Priority : **31.08.87 SE 8703360**

(43) Date of publication of application :
**08.03.89 Bulletin 89/10**

(45) Publication of the grant of the patent :
**22.07.92 Bulletin 92/30**

(84) Designated Contracting States :
**AT BE CH DE ES FR GB IT LI LU NL SE**

(56) References cited :
**EP-A- 0 254 791
DE-A- 3 523 454
US-A- 4 146 653**

(73) Proprietor : **NICASYSTEM AKTIEBOLAG
Neongatan 10
S-431 53 Mölndal (SE)**

(72) Inventor : **Appelgren, Curt Henry
PL 1461 Benjaminssons väg
S-434 00 Kungsbacka (SE)**
Inventor : **Odda, Ulf Andreas
Kastellgatan 15
S-413 07 Göteborg (SE)**

(74) Representative : **Inger, Lars Ulf Bosson
L + U INGER Patentbyra AB Garvaregatan 12
S-262 63 Ängelholm (SE)**

EP 0 306 465 B1

Jouve, 18, rue Saint-Denis, 75001 PARIS

## Description

Technical field

The present invention relates to granular products comprising a heat sensitive organic material of the group of cultures of Lactobacillus, Streptococcus thermophilus, and enzymes.

The object of the present invention is to obtain granular products, which encapsulate and protect heat sensitive and/or moisture sensitive material contained therein in the form of enzymes, and living microorgan-isms.

Background of the invention.

At the production of the granulate the powder is provided with a solvent, optionally containing a binding agent to the formation of a semi moist mass, which is then formed into agglomerates and is dried to granules, or if a suitable size of the agglomerate is obtained directly, is immediately dried to a suitable particle size which varies dependent on the intended use. Optionally, the granulate has to be crushed to obtain the right particle size and particle size distribution. The size may vary from some 100 μm to 1.5 mm in diameter.

All products are, however, not suited for a treatment using a solvent and often an inert carrier has to be added to obtain a suitable granulation.

One way of obtaining a solvent free granulation is to add another powder having good binding properties, to compress the pulverulent mixture into tablets or briquttes and then to crush these into a suitable particle/granulate size.

It shall be noted that a coating with a melted or softened material is previously known. Thus it is known to introduce, into a fluid bed apparatus, a powder and a wax and to obtain a wax coating by increasing the temperature of the air used to be blown through the bed. It is also known to add a powder to a melt present in a vessel provided with a jacket, to allow the melt to stiffen, and to crush it to a suitable size. These processes, however, leads to high increases of temperature in the material to be treated for a long time.

It is previously known to improve the stability of stabilized vitamins using a fat in solid state so that the fat is being absorbed onto the surface of the vitamin particles, (EP-A1-0 125 894). The same patent specification also discloses that vitamin particles which are heat sensitive can not be coated using a heated, molten coating material.

It is further known from US-A-4,146,153 to coat pellets containing vitamins with a melt of xylitol/sorbitol in order to mask bad taste.

It is further known to add cultures of Lactobacillus to the gastro-intestinal tract in order to improve the microorganism flora of the gastro intestinal tract. Normally the microorganism culture is added in the form of a freeze-dried (lyophilized) product. It has howver, turned out that the amount of living Lactobacillus that passes out into the intestinal tract from the ventricle is often too small to give the effect desired when the environment of the ventricle kills 99% of the micro organisms.

Description of the present invention

It has now surprisingly been shown possible to be able to solve this problem by means of the present invention which is characterized in that it consists of a solid product of the said heat sensitive material, which has been provided with a coating of a melt of fat, xylitol, laktitol, paraffine, fatty acid ester, polyethylene glycole and/or stearic acid.

The coating using a melt of the type indicated shall thereby be carried out under such conditions that the temperature of the solid product is not considerably increased, i.e., does not increase more than 5-10°C for a longer time period, i.e., more than 30 sec.

Momentarily, the temperature increase might be greater, but due to a rapid treatment the granules obtained are chilled in such a way that the increase of the temperature during at most 30 sec. extends to 5-10°C only.

The relation between solid material: melt is 70-90:30-10. The melt has in general a temperature of 10-20°C above the melting point of the product.

Solid, heat sensitive products are enzymes, and Lactobacillus cultures (acidophilus, bulgaricus), Streptococcus thermophilus.

The protection obtained protects the sensitive product against gastric juice, moisture, oxidation, and decomposition by heat.

The coating of the solid product can be done in an apparatus described in S-E-C-7903053-2 and in such a way as described in SE-A-8500487-7.

The apparatus according to SE-C-7903053-2 comprises a mixing house provided with a cover. Within the housing a disc is arranged which disc consists of an upper disc and a lower disc, which disc is rotatably mounted around a shaft by means of a bearing. Between the upper and the lower discs there is a hollow space which is connected to the upper side of the upper disc via an annular slot consisting of two parts which both take the shape of a circumferential surface of a cut cone with its point directed downwardly. The upper disc has blades cut on its upper side and optionally cut, or mounted blades on its side underneath. Between the blades spaces exist which are intended to receive particles to be coated while said space optionally being divided into compartments by means of said blades is intended to receive the melt which is to be used for coating. The lower disc is provided with an edge extending around it close to the opening of the slot. At its periphery the lower disc is provided with blades for throwing out the final product via a diffusor and an outlet. The cover is provided with a compartment placed above the centre of the disc which compartment is arranged to receive the particulate solid material for a further transport thereof to said above mentioned spaces between the blades. The particulate solid material is fed to the compartment of the cover by means of a transporting device, e.g. a feeding screw. The liquid phase is being fed to the space via a tube.

At the coating operation according to SE-A-8500487-7 the discs are rotated within the housing around the shaft with a revolving speed of between 1000 to 500 rpm. A suitable peripheral speed using a disc diameter of 300 mm is 1500 to 5000 m per minute whereby the lower speed is used for embedment/agglomeration and the higher for coating. The coating melt is thereby fed to the apparatus via the tube to the hollow space between the upper and the lower discs. The tube is thereby provided with heating means in order to keep the material in melted, liquid form. By means of the centripetal force and the blades the melt is thrown outwardly through the slot and further through the outer slot. The melt hereby takes the form of a membrane which extends outwardly all the time simultaneously as it becomes thinner. When the melt leaves the outer slot the membrane is torn up into very small droplets when it leaves the edge, whereby a mist curtain of droplets having a microscopic size are formed. Simultaneously with the addition of melt through the tube a particulate material is added by means of the transporting device to the compartments and further on to the spaces on the upper side of the upper disc. From there the particulate material is thrown, by means of the centripetal force, and the blades, outwardly towards the periphery of the upper disc, simultaneously as it is deagglomerated into primary particles which meet with and pass the mist curtain at the edge. The particles hereby obtain a surface coating of the melt and are further thrown outwardly to the blades arranged at the periphery of the lower disc, which blades throw the product out off the apparatus via the diffusor and the outlet. Depending on the number of blades their height and the through-put, which can be varied using the outlet area, one can get an agglomeration of the particles, or get the particles out as separate sole, coated particles. The granulate obtained is directly chilled with air during the throwing out thereof and is further chilled at a following separation in a cyclone or the like.

Example 1

850 g of a mixture of 80% mannitol, 10% skim milk powder and 10% lyophilized culture of Lactobacillus acidophilus were introduced into an apparatus according to the above in the form of a powder, whereby a melt of stearic acid having the temperature of 70°C was introduced in an amount of about 200 g, which in the contact zone between powder and melt coated the powder with a layer of the hardening melt. The procedure was repeated further twice to a final content of Lact. acidophilus of 6.2% in the final product.

At trials carried out the degree of survival was 85% at pH 1.2.

Example 2

A pulverulent composition of 10% of Lact. acidophilus, 87% of mannitol, and 3% of microcrystalline cellulose was coated with a fatty acid ester (Brij 98) once at 50°C. The degree of survival obtained was 92% at pH 1.2, 1 hr.

Example 3

A pulverulent composition of 10% of Lact. acidophilus, 80% of dicalciumphosphate and 10% of skim milk powder was granulated four times using stearic acid at 70°C using about 210 g of stearic acid each time to a final content of Lact. acidophilus of 5.4%. The degree of survival was 100% at pH 1.2, 1 hr.

Example 4

100 g of pure Lactobacillus acidophilus culture, lyophilized, and 900 g of pulverulent stearic acid were introduced as a powder in an apparatus as mentioned above, and were coated with a melt of stearic acid in two steps using 32.5% of the final product. Final content of bacteria was 6.8%. 38% of the ingoing bacteria survived the process and 12% of the bacteria survived 1 hr at pH 1.2.

Example 5

300 g of Lactobacillus acidophilus culture, lyophilized, and 700 g of pulverulent stearic acid were mixed and introduced in an apparatus as mentioned above, and were coated with a melt of stearic acid in two steps to a final content of 32.5% of melt. The final product contained 20.3% Lact. acidophilus. The degree of survival was 100 through the process and was 46.7 after 1 hr at pH 2.

Example 6

400 g of Lact. acidophilus culture, lyophilized, and 600 g of pulverulent stearic acid were mixed and coated with a melt of stearic acid to a final content of melt of 32.5% of stearic acid. The final content of bacteria in the product was 27.9%. The degree of survival of the process was 91%, and was 42% after 2 hrs at pH 1.2.

## Claims

1. A granular product comprising a heat sensitive organic material of the group of cultures of Lactobacillus, Streptococcus thermophilus, and enzymes **characterized** in that it consists of a solid product of the said heat sensitive material, which has been provided with a coating of a melt of fat, xylitol, laktitol, paraffine, fatty acid ester, polyethylene glycole and/or stearic acid.

2. A granular product according to claim 1, **characterized** in that the solid product is 70-90%, and the solid, previously melted product material is 30-10%, preferably 20-25%..

3. A granular product according to claims 1-2, **characterized** in that a solid material containing a living culture of Lactobacillus is coated with stearic acid and/or a fatty acid ester in the relationship 70-90:30-10.

## Patentansprüche

1. Körniges Produkt, umfassend ein hitzeempfindliches Material aus der Gruppe von Kulturen von Lactobazillus, Streptokokkus thermophilus und Enzymen, dadurch **gekennzeichnet**, daß es aus einem festen Produkt des genannten hitzeempfindlichen Materials besteht, welches mit einer Beschichtung einer Schmelze von Fett, Xylit, Laktit, Paraffin, Fettsäureester, Polyethylenglykol und / oder Stearinsäure versehen wurde.

2. Körniges Produkt, nach Anspruch 1, dadurch **gekennzeichnet**, daß der Anteil an festem Produkt 70 - 90% ist und an festem, vorher geschmolzenem Produktmaterial 30 - 10%. vorzugsweise 20 - 25%, beträgt.

3. Körniges Produkt, nach den Ansprüchen 1 - 2, dadurch **gekennzeichnet**, daß ein festes Material, das eine lebende Kultur von Lactobazillus enthält, mit Stearinsäure und / oder Fettsäureester im Verältnis 70-90:30-10 beschichtet ist.

## Revendications

1. Produit granulaire comprenant une matière organique thermosensible appartenant au groupe comprenant les cultures de Lactobacillus, le Streptococcus thermophilus, et les enzymes, **caractérisé** en ce qu'il consiste en un produit solide de cette matière thermosensible, qui a été pourvu d'un enrobage d'une masse fondue d'une graisse, de xylitol, de lactitol, de paraffine, d'ester d'acide gras, de polyéthylène glycol et/ou d'acide stéarique.

2. Produit granulaire suivant la revendication 1, **caractérisé** en ce que le produit solide constitue de 70 à 90% et la matière solide, préalablement fondue, constitue 30 à 10%, de préférence 20 à 25%.

3. Produit granulaire suivant la revendication 1 ou 2, **caractérisé** en ce qu'une matière solide contenant une culture vivante de Lactobacillus est pourvue d'un enrobage par de l'acide stéarique et/ou un ester d'acide gras dans le rapport de 70-90:30-10.